Europäisches Patentamt

European Patent Office

Office européen des brevets

(11) Veröffentlichungsnummer : **0 136 309**
**B1**

(12) # EUROPÄISCHE PATENTSCHRIFT

(45) Veröffentlichungstag der Patentschrift :
06.05.87

(21) Anmeldenummer : 84900857.8

(22) Anmeldetag : 23.02.84

(86) Internationale Anmeldenummer :
PCT/EP 84/00046

(87) Internationale Veröffentlichungsnummer :
WO/8403274 (30.08.84 Gazette 84/21)

(51) Int. Cl.⁴ : **C 01 B 11/00**, A 01 N 59/00,
A 01 N 25/22

(54) **VERFAHREN ZUR HERSTELLUNG EINER MODIFIZIERTEN WÄSSRIGEN CHLORITLÖSUNG, DIE DANACH HERGESTELLTE LÖSUNG SOWIE DEREN VERWENDUNG.**

(30) Priorität : 25.02.83 DE 3306753
03.03.83 DE 3307569
02.02.84 DE 3403631

(43) Veröffentlichungstag der Anmeldung :
10.04.85 Patentblatt 85/15

(45) Bekanntmachung des Hinweises auf die Patenterteilung : 06.05.87 Patentblatt 87/19

(84) Benannte Vertragsstaaten :
AT BE CH FR GB LI LU NL SE

(56) Entgegenhaltungen :
FR-A- 959 544
US-A- 2 358 866
Die Akte enthält technische Angaben, die nach dem
Eingang der Anmeldung eingereicht wurden und die
nicht in dieser Patentschrift enthalten sind.

(73) Patentinhaber : BERGER, Peter
Rathausstrasse 44
D-6900 Heidelberg (DE)

(72) Erfinder : BERGER, Peter
Rathausstrasse 44
D-6900 Heidelberg (DE)

(74) Vertreter : Hagemann, Heinrich, Dr. et al
Patentanwälte GEYER, HAGEMANN & KEHL Postfach
860329
D-8000 München 86 (DE)

## Beschreibung

Die Erfindung betrifft ein Verfahren zur Herstellung einer stabilisierten, modifizierten, alkalisch eingestellten wässrigen Chloritlösung sowie die Verwendung der danach erhaltenen wässrigen Chloritlösung als Biozid.

Modifizierte, alkalisch eingestellte wässrige Chloritlösungen, insbesondere insbesondere auch mittels Peroxyverbindungen stabilisierte Natriumchloritlösungen, finden in der Technik wie auch in anderen Bereichen vielfältige Verwendung. Aufgrund ihrer oxidativen Wirkung werden sie u. a. zur Aufbereitung von Wasser zu Trinkwasserzwecken sowie von Schwimmbadwasser und auch von Betriebswasser zum Entkeimen herangezogen. Da diese Lösungen nicht stets unmittelbar nach ihrer Herstellung, sondern häufig erst nach längerer Lagerzeit eingesetzt werden, spielt ihre Stabilisierung eine wesentliche Rolle. Bekannt ist die Verwendung alkalischer Stabilisierungsmittel, wie z. B. Natriumcarbonat, und von Wasserstoffperoxyd und davon abgeleiteten anorganischen Verbindungen, wie z. B. Perboraten. Derartig stabilisierte, modifizierte, alkalisch eingestellte wässrige Chloritlösungen ergeben sich beispielsweise aus der DE-C-2 728 170 und der DE-C-2 730 883.

Die US-A-2 358 866 beschreibt eine sauer eingestellte stabile Chloritlösung. Die Stabilisierung erfolgt durch die Einverleibung von Wasserstoffperoxid, wodurch die Bildung von Chlordioxid unterdrückt werden soll. Die Stabilisierung wird nur in einem sauren Medium erreicht. Saure Lösungen sind jedoch in verschiedenen Anwendungsgebieten nachteilig oder nicht einsetzbar. Das gilt beispielsweise bei der Behandlung von Hautkrankheiten.

Es ist bekannt, daß wässrige Chloritlösungen, in denen im sauren Bereich Chlordioxid mit Chlorsäure im Gleichgewicht steht, mittels Pyridin stabilisiert werden können (vgl. Holleman/Wiberg « Lehrbuch der anorganischen Chemie », 47-56. Aufl., 1960, S. 127). Mit Pyridin stabilisierte Chloritlösungen lassen sich nicht in Systemen einsetzen, in denen eine zusätzliche Belastung mit organischer Substanz vermieden werden muß, wie z. B. im Bereich der Aufbereitung von Trinkwasser und Schwimmbadwasser. Unter anderem ist Pyridin ein karzionogener Stoff.

Ein besondere Rolle spielen stabilisierte oder auch nicht stabilisierte Chloritlösungen bei der Behandlung von Schwimmbadwasser. Herkömmliche Chloritlösungen, auch stabilisierte Chloritlösungen, müssen bei einem derartigen « Chlor/Chlordioxid-Verfahren » eine saure Reaktionsstrecke bei einem pH-Wert ≤ 3 durchlaufen. Hierfür sind zwingend gesonderte und nicht dem Wasserzyklus unmittelbar integrierte Apparaturen vorgeschrieben. Das gilt sowohl für das Säure-Verfahren als auch für das Unterchlorige-Säure-Verfahren, die beide dem obengenannten Chlor-/Chlordioxid-Verfahren unterzuordnen sind. Werden hierbei Natriumchlorit und Salzsäure eingesetzt, dann werden nach diesem sogenannten Salzsäure-Verfahren bei dem pH-Wert von ≤ 3 aus Natriumchlorit und Salzsäure Natriumchlorid und Chlordioxid gebildet. Hierfür werden Reaktionstürme mit Raschig-Ringen in einer Größe empfohlen, die eine entsprechende Reaktionszeit gewährleisten, um eine möglichst hohe Umsetzungsrate zu erzielen. Damit soll erreicht werden, daß bei Einleiten des Reaktionsproduktes der Restgehalt an Chlorit im Schwimmbeckenwasser möglichst gering ist, insbesondere höchstens 0,1 mg/l beträgt. Um eine Redisproportionierung zu Chlorit-Chlorat zu verhindern, werden die Verhältnisse von Chlor zu Chlordioxid im Schwimmbeckenwasser mit 10 : 1 vorgeschrieben.

Bei der Durchführung des Unterchlorige-Säure-Verfahren werden beispielsweise eine Natriumchloritlösung sowie Chlor in einen Wasserbehälter, der der Stelle der Wasserdesinfektion vorgeschaltet ist, dosiert. Die gewünschte Reaktion läuft bei einem pH-Wert von ≤ 3 ab, bei der als Zwischenstufe unterchlorige Säure und schließlich Chlordioxid gebildet werden. Die auf diese Weise hergestellte Lösung wird nach Bedarf dem Beckenwasser zudosiert.

Bei beiden obenbeschriebenen Verfahren ist ein hoher Aufwand an Geräten erforderlich und stets darauf zu achten, daß bei dem Säure-Verfahren die Salzsäurezufuhr und bei dem Unterchlorige-Säure-Verfahren die Chlorzufuhr gewährleistet ist, um eine nachteilige Zudosierung nicht umgesetzten Chlorits in das Beckenwasser zu vermeiden. Da hier gelegentliche Störungen nicht sicher auszuschließen sind, ergibt sich eine stetige Gefährdung des Badegastes. Darüber hinaus kann das zufällige Vermischen handelsüblicher Chloritlösungen mit Säuren zu explosionsartigen Erscheinungen führen.

Der Erfindung lag die Aufgabe zugrunde, das eingangs beschriebene Verfahren derart zu verbessern, daß ein im sauren Medium stabileres Verfahrensprodukt hergestellt werden kann, das gefahrlos und mit größerer Effizienz zur Aufbereitung von Wasser, insbesondere Schwimmbadwasser, herangezogen werden kann und bessere Hautverträglichkeiten zeigt.

Erfindungsgemäß wird diese Aufgabe dadurch gelöst, daß eine einen pH-Wert ≤ 3 aufweisende, wässrige, sulfationenhaltige Lösung mit einer darin stabilen Peroxyverbindung versetzt wird, die eine Konzentration an Peroxyverbindung im Endprodukt von etwa 0,001 bis 0,01 molar ergibt, und diese Lösung anschließend mit der wässrigen, alkalischen Lösung eines Chlorits in einer Menge vermischt wird, daß ein pH-Wert von über 7,0, insbesondere zwischen 7,5 und 8,0, eingestellt wird.

Vorzugsweise wird als Peroxyverbindung eine anorganische Peroxyverbindung in Form von Wasserstoffperoxyd, eines Persulfats, Percarbonats, Perborats oder eines Peroxids eines Alkali-oder Erdalkalimetalls verwendet. Als Chlorit wird vorzugsweise ein Alkali- und/oder Erdalkalichlorit eingesetzt. Besonders vorteilhafte Ergebnisse stellen sich dann ein, wenn die wässrige, sulfationenhaltige Lösung einen pH-Wert $\leq 1$ aufweist.

Die bei der Durchführung des erfindungsgemäßen Verfahrens eingesetzten Peroxyverbindungen dienen dazu, das erhaltene und auf Chlorit basierende Oxidationssystem langzeitig unter Beibehaltung der angestrebten oxidativen Wirksamkeit zu stabilisieren. Der Begriff « Peroxyverbindungen » soll weitestgehend verstanden werden. So fallen darunter Wasserstoffperoxid ($H_2O_2$) sowie Derivate des Wasserstoffperoxids, insbesondere Peroxyverbindungen (vorzugsweise in Form anorganischer Verbindungen), die das Ion $O_2^{2-}$ als Ligand enthalten oder in denen —O— durch —O—O— ersetzt ist, Peroxide und Persäuren sowie deren Salze. Zu den Peroxyverbindungen zählen insbesondere anorganische Peroxysäuren und, wie bereits erwähnt, deren Salze. Für die Zwecke der Erfindung kommen auch Oxosäuren vom Typ der Perchlorsäure, einschließlich deren Salze, in Frage. Unter den Salzen obiger Säuren stehen die Natrium-, Kalium- und Calciumverbindungen im Vordergrund. Werden zur Stabilisierung Peroxide herangezogen, dann sind dies vorzugsweise Natrium- oder Bariumperoxid.

Für den Fall, daß die erfindungsgemäß hergestellte wässrige Chloritlösung in Systemen eingesetzt wird, in denen zusätzlich eingeführte organische Substanzen unbedenklich sind, können obige Peroxyverbindungen auch organischer Natur sein. Im Rahmen der gegebenen Lehre wird der Fachmann noch weitere zur Stabilisierung der wässrigen Chloritlösung geeignete Peroxyverbindungen ermitteln können.

Es muß überraschen, daß für die Zwecke der Erfindung verhältnismäßig geringe Konzentrationen an Peroxyverbindung ausreichen und bei der Anwendung der erhaltenen stabilisierten Verfahrenserzeugnisse zu besonders günstigen Effekten führen. Dies gilt für die 0,001 bis 0,01 molare Konzentration der Peroxyverbindung in der fertigen stabilisierten Lösung, die eine grünliche Färbung aufweist. Höhere Konzentrationen bringen im Hinblick auf die Zielsetzung der Erfindung keinerlei Vorteile und schließen die angestrebten Effekte häufig aus bzw. lassen sie nicht in dem angestrebten Maße erzielen. Eine geringe Menge an stabilisierender Peroxyverbindung bedeutet darüber hinaus praktisch keinen Mehraufwand an Kosten.

Bei der Durchführung des erfindungsgemäßen Verfahrens kann im einzelnen wie folgt vorgegangen werden : eine geeignete stabilisierende Peroxyverbindung wird in eine schwach saure sulfationenhaltige Lösung überführt. Diese Lösung hat einen pH-Wert von $\leq 3$ wobei ein pH-Wert $\leq 1$ ganz besonders vorteilhaft ist. Die Einstellung dieses pH-Wert kann durch Ansäuern mittels Mineralsäuren, wie Salzsäure und Schwefelsäure, aber auch durch Zugabe von Hydrogenverbindungen, d. h. von sauren organischen Salzen, insbesondere Alkali- oder Erdalkalimetallsalzen, oder in Form einer Kombination obiger Verbindungen erfolgen. Natrium-, Kalium- und Calciumsalze, insbesondere in Form ihrer Hydrogensulfate, werden hierbei bevorzugt.

Bei der Auswahl der ansäuernden Verbindungen sollte auch der spätere Verwendungszweck des erfindungsgemäß erhaltenen Verfahrenserzeugnisses berücksichtigt werden. Im allgemeinen ist es von besonderem Vorteil, wenn die Sulfationen durch Schwefelsäure gebildet werden. Es kann hier beispielsweise auch so vorgegangen werden, daß ein Sulfat beigegeben und der gewünschte pH-Wert mit einer anderen Säure eingestellt wird. Das Ansäuern könnte grundsätzlich auch mit Salzsäure erfolgen. Hierbei stellt sich jedoch gegenüber dem Einsatz von Schwefelsäure ein gewisser Nachteil dahingehend ein, daß zu große Mengen an Chlorid trotz der geschilderten Wechselwirkung mit Chlorit vorzeitig und nicht erst im Bedarfsfalle Chlordioxid entstehen lassen, das dann das gewünschte Gleichgewichtssystem unter Ausgasen und damit durch Entzug von Chlordioxid stört. Dies führt zu einer unerwünschten Schwächung des Systems im Sinne der Erfindung.

Der oben beschriebenen sauren wässrigen Lösung der Peroxyverbindung wird eine wässrige Chloritlösung zudosiert, wobei deren Konzentration keine entscheidende Rolle spielt. So kann es sich dabei insbesondere um eine handelsübliche wässrige Alkali- und/oder Erdalkalichloritlösung handeln, insbesondere um eine Natrium- und/oder Calciumchloritlösung. Der pH-Wert der handelsüblichen Alkalichloritlösung liegt im allgemeinen über 12. Lösungen dieser Art enthalten regelmäßig etwa 300 g/l Chlorit. Selbstverständlich kann dieser Wert auch unterschritten werden. Da erfindungsgemäß regelmäßig eine stark konzentrierte stabilisierte und modifizierte Chloritlösung angestrebt wird, wird man selbstverständlich auch eine stark konzentrierte Chloritlösung als Ausgangsmaterial heranziehen. Die Zudosierung der Ausgangschloritlösung erfolgt vorzugsweise unter Rühren, bis die ursprünglich teilweise gasend dunkelbraune Lösung einen grünlich-gelben bzw. grünlichen Farbton angenommen hat. Dieses ist dann der Fall, wenn der pH-Wert über 7 liegt. Bevorzugt wird hier ein pH-Wert von etwa 7 bis 8, insbesondere 7,5 bis 8.

Überraschenderweise hat es sich gezeigt, daß bei dem erfindungsgemäßen Verfahren die Menge an Peroxyverbindung herabgesetzt werden kann, wenn der Fertiglösung ein wasserlösliches Phosphat, so z. B. Natriummetapolyphosphat, in geringer Menge, einverleibt wird.

Eine übermäßige Reaktion kann bei dem erfindungsgemäßen Verfahren in Form explosionsartiger Erscheinungen dann eintreten, wenn die eingesetzten wässrigen Ausgangslösungen lediglich eine geringe Carbonathärte zeigen bzw. es sich um demineralisiertes Wasser handelt. In einem derartigen Falle empfiehlt es sich, unter einer Inertgasatmosphäre zu arbeiten. Es wird bevorzugt, der sauren wässrigen Lösung vor dem Zudosieren der Chloritlösung eine in der Regel ausreichende geringe Menge eines Hydrogencarbonats, z. B. Natriumhydrogencarbonats, zuzudosieren. Bei wässrigen Ausgangslösungen mittlerer bzw. hoher Carbonathärte ist eine derartige Maßnahme im allgemeinen nicht nötig, da sich eine Art Schutzgasschicht aus Kohlendioxid zwischen dem geringfügig ausgasenden Chlordioxid und der Luft ausbildet. Damit kann kein explosives Luft/Chlordioxid-Gemisch entstehen. Ein derartiges Gemisch neigt bei einem Verhältnis der Bestandteile Luft zu Chlordioxid von etwa 10 : 1 zu einem explosionsartigen Zerfall des Chlordioxids in Chlor und Sauerstoff.

Die nach dem erfindungsgemäßen Verfahren hergestellte Chloritlösung zeigt eine außergewöhnlich günstige Lagerzeit von mehreren Monaten, ohne daß die gewünschte oxidative Wirkung bedeutsam herabgesetzt wird. Nach einiger Zeit stellt sich eine gelb-braune Färbung durch anteilig freigesetztes Chlordioxid ein, wodurch jedoch die Wirksamkeit nicht wesentlich beeinträchtigt wird. Entzieht man dieser wässrigen Lösung den freigelösten Chlordioxid-Anteil, z. B. durch Ausschütteln mit Tetrachlorkohlenstoff, dann tritt nach kurzer Zeit die erwähnte grünliche Färbung der klaren wässrigen Lösung wieder auf. Das ist so zu deuten, daß die Chloritlösung im Sinne einer Gleichgewichtsreaktion stets Chlordioxid bei dessen Entzug nachbildet. Diese Nachbildung tritt selbst in großer Verdünnung ein, so z. B. auch dann, wenn das gebildete Chlordioxid seine oxidative Wirkung durch den Einfluß reduzierender Substanzen, insbesondere organischer Substanzen verbraucht hat.

In der erfindungsgemäß hergestellten Chloritlösung stellt sich, im Gegensatz zu den üblichen Chloritlösungen des Standes der Technik, ein positives Redoxpotential je nach Lichteinwirkung zwischen etwa 450 bis 500 mV ein. Für die spätere Wirkungsweise sowie für die nachfolgend näher beschriebenen Wirkungsbereiche ist es wesentlich, daß mit einer möglichst kleinen und kontrollierbaren Menge einer Peroxyverbindung ein stabilisierender Effekt erreicht wird. Da die stabilisierende Wirkung von Peroxyverbindungen gegenüber Chlordioxid bzw. Chlorit-Lösungen stark pH-abhängig ist, muß nach bekanntem Vorgehen eine relativ große Menge an Peroxyverbindung einer zu stabilisierenden sauren Chlordioxidlösung beigegeben werden. Entsprechendes gilt auch für die Stabilisierung im alkalischen Bereich. Hier müssen sogar große Überschußmengen an Peroxyverbindung aufgrund der Tatsache zugegeben werden, daß sich diese im alkalischen Bereich stark zersetzt. Hierbei läßt sich nach der pH-Reduzierung nicht mehr ermitteln, wieviel Peroxyanteile noch in der fertigen Lösung vorhanden sind. Grundsätzlich stören jedoch größere Anteile an Peroxyverbindungen bei der späteren Anwendung der beiden zuletzt beschriebenen und dem Stand der Technik zuzurechnenden Chloritlösungen in den eingangs bereits beschriebenen Anwendungsbereichen in einem nicht akzeptablen Maß. Zum anderen sollte eine Chloritlösung bei bestimmten Anwendungsbereichen möglichst etwa pH-neutral eingestellt sein, was für die oben beschriebenen und entsprechend dem Stand der Technik hergestellten Lösungen, im Gegensatz zu der erfindungsgemäßen Chloritlösung, nicht gilt, deren technologischer Hintergrund durch die nachfolgenden Ausführungen im Hinblick auf den geschilderten Stand der Technik noch deutlicher gemacht werden soll.

Bei dem erfindungsgemäßen Verfahren wird beispielsweise wie folgt vorgegangen : einer vorgegebenen sauren sulfationenhaltigen (insbesondere schwefelsauren) Lösung, deren pH-Wert vorzugsweise $\leq 1$ ist, wird eine sehr geringe Menge an Peroxyverbindung zugegeben, so daß sich in der fertigen Chloritlösung eine Konzentration von etwa 0,002 2 Mol Peroxyverbindung einstellt. Diese fertige Lösung enthält zirka 100 g Chlordioxid pro 1. Um eine derartige Menge Chlordioxid mit möglichst geringen Mengen an Peroxyverbindung im pH-Bereich von 7 bis 8 zu stabilisieren, wird wie folgt verfahren : zirka 200 l Leitungswasser einer Carbonathärte von 18 werden ca. 6 kg eines perlierten Natriumhydrogensulfat unter Rühren einverleibt. Dieser Lösung werden zirka 100 ml einer 30 %igen $H_2O_2$-Lösung zugegeben. Nach ca. 10-minütigem Rühren werden dieser Lösung während etwa 10 min 200 l einer ca. 30 %igen Natriumchloritlösung bis zum Erreichen des Neutralpunktes zugeführt. In der noch stark sauren Phase reicht die geringe Menge Peroxyverbindung aus, um den noch geringen Anteil an Chlordioxid stabil zu halten. Da mit steigendem pH-Wert der stabilisierende Charakter der Peroxyverbindung gegenüber Chlordioxid zunimmt, verlaufen bei diesem Vorgehen die Prozesse entsprechend einem sich selbst steuernden Äquivalent sauber ab. Es entsteht möglicherweise eine komplexe Verbindung des Chlordioxids, bei der die Peroxyverbindung nicht wie bei anderen bekannten Verfahren in den chemischen Reaktionsmechanismus eingreift (wie z. B. entsprechend der Gleichung $2 \ ClO_2 + 2 \ HO^- + H_2O_2 \rightarrow 2 \ ClO_2^- + 2 \ H_2O + O_2$). Bei dem Erreichen eines pH-Wertes von ca. 7 ändert die vorher dunkelbraune Lösung ihre Farbe nach lindgrün. Diese Lösung weist ein Oxidationspotential von etwa 300 mV auf. Setzt man diese Lösung ca. 24 Std. direktem oder indirektem Tageslicht aus, dann findet nach einer gewissen Zeit eine sogenannte Kickreaktion statt. Die Lösung nimmt darauf eine goldgelbe Farbe an. Hiermit ist gleichzeitig ein Potentialsprung auf ca. + 450 bis 550 mV verbunden.

Behandelt man nun die in oben beschriebener Weise hergestellte Lösung mit Tetrachlorkohlenstoff, dann wird hiermit der Anteil an freiem Chlordioxid ausgeschüttelt. Das freie Chlordioxid geht unter Gelbfärbung in die Tetrachlorkohlenstoffphase über. Die wässrige Phase ist hell. Nach kurzer Zeit läßt sich beobachten, daß in der verbliebenen wässrigen Phase erneut Chlordioxid nachgebildet wird (Gelbfärbung). Dieser Vorgang kann bis zur Erschöpfung der wässrigen Phase wiederholt werden. Wird die erfindungsgemäße stabilisierte Chloritlösung in warmes Wasser gegeben, dann stellt sich neben dem typischen chlorähnlichen auch ein leichter Ozongeruch ein.

Wird die erfindungsgemäß erhaltene Chloritlösung mit Wasser stark verdünnt, dann verschiebt sich das in ihr vorliegende Gleichgewicht in Richtung des Chlordioxids, was auch noch in dem pH-Bereich von etwa 7 bis 8,5 gilt.

Wird die erfindungsgemäß erhaltene Chloritlösung in der Praxis verwendet, dann tritt die gewünschte Wirkung der neutralen bzw. schwach alkalischen Lösung durch Ansäuern oder unter Einwirkung eines beliebigen Reaktionspartners, so z. B. Chlor, ein, wodurch Chlordioxid freigesetzt wird, das unter Normalbedingungen ein gelblich-rötliches Gas darstellt. Durch das Vorhandensein von Chlor, insbesondere bei erhöhtem Chloridanteil, oder Mineralsäuren in einem behandelten wässrigen System läuft die Reaktion der Chlordioxidbildung gegenüber der unerwünschten Chlorats vorrangig ab. Der gewünschte Reaktionsablauf wird selbst bei Zugabe starker Mineralsäuren in hoher Konzentration nicht mehr, anders als bei den bekannten Chloritlösungen, gestört. Letztere zeigen nämlich unter Einwirkung starker Mineralsäuren weiterhin die bereits erwähnten explosionsartigen Erscheinungen.

Andererseits gibt es auch Fälle, in denen die erfindungsgemäß erhaltene Chloritlösung einer Destabilisierung unterliegt, indem sich in dem zu behandelnden System für die in kleiner Menge vorliegende Peroxyverbindung ein reaktionspartner findet, der diese reduziert und damit die Stabilisierung der Chloritlösung aufhebt. Bei einem derartigen Reaktionspartner kann es sich z. B. um die in diversen Keimzellen vorhandenen Peroxydasen und Katalasen handeln, d. h. um Enzyme des pflanzlichen und tierischen Stoffwechsels, die zur Gruppe der Peroxidoreduktasen gehören. Durch deren Einwirkung wird spontan Chlordioxid freigesetzt. Diese Tatsache verleiht der stabilisierten modifizierten Chloritlösung im bioziden Einsatz bei geringen Dosiermengen hohe Selektivität.

Mit besonderem Vorteil läßt sich die erfindungsgemäß erhaltene Chloritlösung, insbesondere in Form einer Natriumchloritlösung, bei der Wasseraufbereitung einsetzen. Dabei steht die Aufbereitung von Trinkwasser, Betriebswasser sowie Schwimmbadwasser zu Entkeimungszwecken im Vordergrund. Diese Entkeimung erfolgt vorzugsweise unter gleichzeitiger Verwendung von Chlor, so daß das für die Entkeimungszwecke stark wirksame Chlordioxid entsteht. Zur Herstellung der stabilisierten Chloritlösung wird z. B. eine Natriumchloritlösung mit ca. 300 g Natriumchlorit pro 1 herangezogen. Die fertige Lösung enthält im allgemeinen umgerechnet 8 bis 15 Gew.-% Chlordioxid, wobei der Bereich von 10 bis 12 Gew.-% unter den verschiedenen Gesichtspunkten regelmäßig von besonderem Vorteil ist. Dieser Lösung können noch verschiedene andere Bestandteile beigegeben werden, wie Natriumchlorid, Natriumsulfat und Natriumchlorat. Die erhaltene Natriumchloritlösung kann durch Verdünnen so eingestellt sein, daß sie die KOK (Koordinationskreis für Bäderbau und Bäderbetrieb)-Richtlinien erfüllt, wobei ein Restgehalt an Chlorit im Schwimmbeckenwasser mit maximal 0,3 mg/l Beckenwasser festgelegt ist. Sie erfüllt dann aber auch den DIN-Entwurf 19643 mit maximal 0,1 mg/l. Die vorstehenden Ausführungen gelten auch für andere Chloritlösungen, wie andere Alkali- und Erdalkalichloritlösungen.

Von besonderem Wert ist die erfindungsgemäß hergestellte Chloritlösung bei der Aufbereitung von Schwimmbadwasser, worauf nachfolgend detailliert eingegangen wird :

Zunächst ist hervorzuheben, daß die bei den eingangs geschilderten bekannten Verfahren eintretenden explosionsartigen Erscheinungen ausgeschlossen werden, wenn die erfindungsgemäß gewonnene Chloritlösung eingesetzt wird. Sie ist selbst bei Vermischen mit konzentrierter Schwefelsäure nicht zur Explosion zu bringen. Ihre besondere Eignung zur Aufbereitung bzw. Konditionierung von Schwimmbadwasser wurde durch ein Gutachten des Hygieneinstituts/Gelsenkirchen nachgewiesen. So zeigte ein Gleichwertigkeitsnachweis, daß sich diese Chloritlösung völlig anders als handelsübliche oder auf andere Weise stabilisierte Chloritlösungen verhält. Bei dem Versuch des Hygieneinstitutes wurde die erfindungsgemäß hergestellte Chloritlösung mittels einer Feindosierpumpe über eine Impfstelle zwischen Flockungsimpfstelle und Filter direkt in den Beckenwasserkreislauf (eines pH-Wertes von 7,5) dosiert. Bei allen gezogenen Wasserproben wurde Chlordioxid, nicht jedoch das zu vermeidende Chlorit ($ClO_2^-$) nachgewiesen. In diesem Zusammenhang ist auch der Hinweis von Bedeutung, daß bereits kleinste Mengen an Peroxyverbindungen in gechlortem Beckenwasser störend wirken. Peroxide nehmen gegenüber den starken Oxidationsmitteln, wie Chlor, reduktiven Charakter an. Sie drücken daher das Oxidationspotential erheblich, das im Schwimmbadwasser als Parameter für die Keimtötungsgeschwindigkeit eine wesentliche Rolle spielt. Nicht zuletzt wird heute bereits in vielen Schwimmbädern die Desinfektionsmittelzugabe über diesen Parameter automatisch gesteuert. Bei einer Künstlichen Veränderung (Reduzierung) des Oxidationspotentials würden Vielfachmengen z. B. an Chlor unkontrolliert in das Badewasser gelangen. Diese Gefahr wird durch den Einsatz der erfindungsgemäßen Chloritlösung weitestgehend ausgeschlossen. Die durch die hiermit in den Beckenkreislauf eingebrachten Mikromengen an Peroxyverbindungen liegen in einer Größenordnung, die gerade noch durch z. B. feinverteilte Metallverunreinigungen auf dem Filter oder in einigen Keimen vorkommende Peroxydase eliminiert werden können. Durch das derartig selektiv in verschmutztem und verkeimtem Filtermaterial spontan freigesetzte Chlordioxid treten zusätzliche Phänomene auf, die bei herkömmlichen Chlor/Chlordioxid-Verfahren vergleichsweise nicht stattfinden.

Des weiteren wurde bei dem erwähnten Gleichwertigkeitsnachweis gezeigt, daß sich der Zeitraum zwischen den nach Differenzdruck ergebenden notwendigen Filterrückspülungen verdoppelte bzw. die zur Rückspülung benötigte Beckenwassermenge um die Hälfte reduziert werden konnte. Des weiteren konnte gezeigt werden, daß die mit der erfindungsgemäßen Chloritlösung behandelten Filter in der halben Zeit die doppelte Menge an Schmutzstoffen austragen. Das geht möglicherweise auf eine bessere Koagulation der organischen/anorganischen Belastungsstoffe une auf eine Verhinderung des Verklebens des Filtermaterials zurück. Hierdurch ist ein leichteres und schnelleres Austragen der eingefangenen Verschmutzungsteilchen möglich. Außerdem verhindert die erfindungsgemäße Chloritlösung das starke Verkeimen hochbelasteter Filter. Demzufolge dient sie in der speziell eingestellten Weise auch als Filterhilfsmittel. Aus ihrem geschilderten Einsatz resultieren erhebliche Einsparungen an Frischwasser-, Abwasser-, Aufheiz- und Aufbereitungskosten. In dem für die Untersuchung des erwähnten Gleichwertigkeitsnachweises gewählten Hallenbad Bockum/Hövel in Hamm stellt sich das wie folgt dar : bei der normalen Aufbereitung Flockung-Filterung-Chlorung liegt die notwendige Frischwassermenge je Badegast bei 0,11 m$^3$, während bei dem entsprechenden Vorgehen mit der erfindungsgemäßen Chloritlösung je Badegast durchschnittlich 0,03 m$^3$ Frischwasser erforderlich sind. Die Einsparung je Badegast betrug demzufolge 0,08 m$^3$.

Im Ergebnis läßt sich zusammengefaßt zum Einsatz der erfindungsgemäß hergestellten Chloritlösung zur Aufbereitung von Schwimmbadwasser folgendes feststellen : diese Lösung kann direkt dem chlorierten Schwimmbadwasser zugesetzt werden, wobei im pH-Bereich von etwa 7 bis 7,8 direkt Chlordioxid gebildet wird. Die Behandlung erfolgt vorzugsweise zwischen Flockungs- und Filterstufe. Hier können z. B. etwa 24 ml einer etwa 10 gew.-%igen Chloritlösung auf etwa 180 m$^3$ umgewälztes Schwimmbadwasser entfallen. Die 100 %ige Umsetzung zu Chlordioxid verläuft dabei so schnell, daß bei rohwasserseitigem Dosieren reinwasserseitig in keinem Falle Chlorit, wohl aber Chlordioxid nachgewiesen werden konnte. Die erzielte Wasserqualität ist derjenigen bekannter Verfahren (Säure-Verfahren bzw. Unterchlorige-Säure-Verfahren) überlegen. Das mit der erfindungsgemäßen Chloritlösung arbeitende Aufbereitungsverfahren ist, wie gezeigt, außergewöhnlich kostengünstig.

Die erfindungsgemäßen stabilisierten, modifizierten Chloritlösungen lassen sich noch weiteren Verwendungszwecken zuführen. Es hat sich gezeigt, daß insbesondere verdünnte Chloritlösungen hervorragende biozide Wirkung im weitesten Sinne zeigen. Das gilt insbesondere für eine verdünnte Natriumchloritlösung, vorzugsweise in einer etwa 0,1 bis 0,5 gew.-%igen Konzentration.

So läßt sie sich zur hygienischen desinfizierenden Körperpflege heranziehen, so z. B. zur Fußpflege in Schwimmbädern, und in Saunen, insbesondere auch bei der Behandlung von Schweißfüßen. Besondere Eignung zeigt sie auch bei äußerlich behandelbaren Hautkrankheiten und -reizungen, insbesondere bei Hautflechten, Schuppenflechten, Ekzem und Lupus, Hautpilzen oder allgemein bei entzündlichen Hautkrankheiten, die durch Bakterien und Protozoen, durch Viren oder Pilze (Kandidamykose, Trichophytie, Pityriasis und Herpes) hervorgerufen werden. Auch Hautkrankheiten im Rachen bzw. Mund, wie Zahnfleischbluten, können, insbesondere mit einer Natriumchloritlösung, behandelt werden. Es handelt sich bei diesen Mitteln also um eine Art Arzneimittel. Darüber hinaus lassen sie sich ganz allgemein als Hautpflegemittel verwerten, die in geringen Konzentrationen dem Badewannenwasser beim Baden zugesetzt werden können. Einige der oben bereits angesprochenen Anwendungsfälle sollen nachfolgend detailliert beschrieben werden.

Zunächst wurde im Pharmabereich festgestellt, daß die erfindungsgemäß erhaltene Chloritlösung in hohem Maße hautverträglich ist. So kann sie der gesunden Haut sogar konzentriert zugeführt werden, ohne daß allergische Reaktionen erkennbar werden. Auf normale Chloritlösungen gleicher Konzentration reagiert die Haut im allgemeinen stark allergisch. So wurde im Gegenteil gefunden, daß eine verdünnte erfindungsgemäße Chloritlösung überraschenderweise Allergien abbaut. In Verdünnungen der konzentrierten Lösung zu Wasser von 1 : 10 wurden sogar spektakuläre antimykotische Wirkungen bestätigt. Ebenso erweist sie sich wirksam bei der Abheilung einer teilweise langjährigen schwersten Neurodermitis. Hier muß die Lösung allerdings stark verdünnt angewandt werden, so etwa 20 bis 30 ml pro Wannenbad mit langsamer Steigerung bis an die Grenze der jeweiligen Verträglichkeit des Patienten.

Ein ganz erstaunliches Phänomen stellt sich bei der Behandlung von Psoriasis (Schuppenflechte) ein. Bei täglichen bis 2-täglichen Bädern (ca. 100 ml je Wannenbad unter Ausschluß anderer Badezusätze) tritt bei zirka 80 % der Psoriatiker ein Ablösen der Schuppen, auch ohne mechanische Einwirkung, nach den ersten Bädern ein. Der Juckreiz läßt im allgemeinen schnell nach. Die erhabenen Hautpartien verflachen und die dunkelrote Färbung weicht einem Blaßrosa. Nach einer dann meist eintretenden Stagnationsphase von etwa 2 bis 3 Monaten normalisieren sich die vorher befallenen Hautpartien. Möglicherweise spielt bei dieser Behandlung die sukzessive Abspaltung geringer Mengen Ozon während des Bebadens eine Rolle (Stoffwechselregulation über den großflächigen Hautkontakt). — Das Bebaden des Körpers sowie der Füße etc. beseitigt anhaltend Schweißgeruch. Weitere nützliche Anwendungsbereiche sind Mundspülungen mit 3 %-iger Lösung, wodurdh Zahnfleischbluten beseitigt wird.

Eine ganz erstaunliche Wirkung wurde auch bei der Behandlung diabetischer Gangräne (offener Beine) festgestellt. So heilte in einem Einzelfall eine Wunde von Daumenbreite und einer Länge von etwa 7 cm bei einer Bebadung mit einer verdünnten erfindungsgemäßen Natriumchloritlösung (Verdünnung mit Wasser im Verhältnis 1 : 100) nach einer Behandlung von ca. 8 Wochen.

Schließlich lassen sich die erfindungsgemäßen Chloritlösungen, vorzugsweise die Natriumchloritlösung, im industriellen Bereich heranziehen, so z. B. bei der Schleimbekämpfung in wässrigen Systemen. Dies gilt auch für die Konservierung von Trinkwasser in verschiedenen Behältern bzw. Tanks, insbesondere auf Schiffen. Dort läßt sich eine solche Lösung sehr wirkungsvoll und anwendungstechnisch problemlos einsetzen. Das behandelte Trinkwasser blieb in geschlossenen Behältern noch monatelang keimfrei. Auch war bei den hier eingesetzten Mengen im ppm-Bereich keine geschmackliche Beeinträchtigung des Wassers festzustellen. Auch bestehen unter toxikologischen Gesichtspunkten keinerlei Bedenken.

Die oxidierende Wirkung der erfindungsgemäßen Chloritlösung kann z. B. auch in weiteren technischen Bereichen genutzt werden, so z. B. in Kombination mit Mineralsäuren oder Chlor. Sie lassen sich daher zur Herstellung von Zellulose, bei der Bleichung von Ölen, Fetten und Wachsen, Leder und zur Desinfektion bzw. Desodorierung von übelriechenden Abfällen und Abwässern einsetzen. Ein besonderer Anwendungsfall ist auch die Stufenbleichung von Zellstoff.

Zusammenfassend läßt sich feststellen, daß die erfindungsgemäß hergestellte stabilisierte modifizierte wässrige Chloritlösung überall dort mit Vorteil eingesetzt werden kann, wo sie, insbesondere aufgrund der Bildung von Chlordioxid, oxidative Wirkung entfalten soll. Dabei kann es sich um tote Materie organischer, insbesondere reduzierender Substanzen handeln, wie auch um lebende Organismen, wie Mikroorganismen und Pilze. So kann man diese Chloritlösung im weitesten Sinne als ein biozid wirksames Mittel mit absoluter Hautverträglichkeit bezeichnen, d. h. es kann als Insektizid, Fungizid und Herbizid dienen, wo zum Teil bisher auch handelsübliche Natriumchloritlösungen, jedoch mit geringem Erfolg verwendet wurden bzw. kaum Eignung zeigten. Ein besonderer Vorteil der erfindungsgemäßen Chloritlösung besteht des weiteren darin, daß sie in der Regel eine 100 %ige Umsetzung des Chlorits in Chlordioxid in einem weiten pH-Bereich ermöglicht. Darüber hinaus ist sie einfach und leicht herzustellen, indem beispielsweise die vorgegebene saure Lösung (vorzugsweise eines pH-Wertes ≤ 1) mit einer konzentrierten handelsüblichen Chloritlösung in einem Volumenverhältnis von etwa 1 : 1 bis zur Einstellung eines knapp über 7 liegenden pH-Wertes ohne großen technischen Aufwand gemischt wird.

Nachfolgend soll die Erfindung anhand eines Beispiels noch näher erläutert werden :

Beispiel

In 1 l sulfathaltiges Wasser (Carbonathärte : 18 Grad) eines pH-Wertes von 0,5 werden 0,5 g einer 30 gew.-%igen Wasserstoffperoxidlösung gegeben. Zu dieser Lösung werden unter gutem Rühren 0,9 l einer handelsüblichen Natriumchloritlösung (etwa 300 g Natriumchlorit/l) hinzugefügt. Dabei durchläuft die Lösung eine braune Färbung, die beim Überschreiten des pH-Wertes von 7 nach Ablauf der Stabilisierungsreaktion in eine helle lindgrüne Farbe umschlägt. Hierbei stellt sich in der Lösung ein pH-Wert von etwa 7,5 ein. Diese Lösung liefert bei der Wasseraufbereitung in einem Hallenbad eine 100 %ige Umsetzung zu Chlordioxid. Die Umsetzung verläuft dabei so rasch, daß reinwasserseitig in keinem Falle Chlorit, wohl aber noch in geringen Mengen Chlordioxid nachgewiesen werden kann. Die Wasserqualität wird deutlich verbessert. Der Frischwasserzusatz pro Badegast kann auf 20 % reduziert werden.

Die in obiger Weise hergestellte wässrige Lösung zeigt schließlich in einer 0,1 bis 0,5 gew.-%igen Konzentration auch eine hervorragende Wirkung bei der Behandlung von Schuppenflechten und Hautpilzen.

## Patentansprüche

1. Verfahren zur Herstellung einer durch eine Peroxyverbindung stabilisierten, modifizierten, alkalisch eingestellten wässrigen Chloritlösung, dadurch gekennzeichnet, daß eine einen pH-Wert ≤ 3 aufweisende, wässrige, sulfationenhaltige Lösung mit einer darin stabilen Peroxyverbindung versetzt wird, die eine Konzentration an Peroxyverbindung im Endprodukt von etwa 0,001 bis 0,01 molar ergibt, und diese Lösung anschließend mit der wässrigen, alkalischen Lösung eines Chlorits in einer Menge vermischt wird, daß ein pH-Wert von über 7,0, insbesondere zwischen 7,5 und 8,0 eingestellt wird.

2. Verfahren nach Anspruch 1, dadurch gekennzeichnet, daß als Peroxyverbindung eine anorganische Peroxyverbindung in Form von Wasserstoffperoxid, eines Persulfats, Percarbonats, Perborats oder eines Peroxids eines Alkali- oder Erdalkalimetalls verwendet wird.

3. Verfahren nach einem der Ansprüche 1 oder 2, dadurch gekennzeichnet, daß als Chlorit ein Alkali- und/oder Erdalkalichlorit eingesetzt wird.

4. Verfahren nach Ansprüchen 1-3, dadurch gekennzeichnet, daß die wässrige, sulfationenhaltige Lösung einen pH-Wert ≤ 1 aufweist.

5. Verfahren nach Ansprüchen 1-4, dadurch gekennzeichnet, daß zur Herstellung der wässrigen, sulfationenhaltigen Lösung mineralisiertes Wasser verwendet wird.

6. Verfahren nach Ansprüchen 1-5, dadurch gekennzeichnet, daß dem Endprodukt ein wasserlösliches Phosphat zugesetzt wird.

7. Verwendung der nach dem Verfahren gemäß Ansprüchen 1-6 erhaltenen wässrigen Chloritlösung als Biozid, insbesondere zur äußerlichen Behandlung von Hautkrankheiten und -reizungen sowie zur Wasseraufbereitung.

## Claims

1. A method for the production of a modified, alkalinically-adjusted, aqueous chlorite solution which is stabilised by a peroxy compound, characterised in that an aqueous, sulphate-ion-containing solution having a pH value $\leq 3$ is mixed with a peroxy compound which is stable therein, which results in a concentration of peroxy compound in the end product of about 0.001 to 0.1 molar, and this solution is then mixed with the aqueous alkaline solution of a chlorite in a quantity such that a pH value of above 7.0, expecially between 7.5 and 8.0, is obtained.

2. A method according to Claim 1, characterised in that an inorganic peroxy compound in the form of hydrogen peroxide, a persulphate, percarbonate, perborate, or of a peroxide of an alkali metal or earth alkali metal is used as peroxy compound.

3. A method according to one of Claims 1 or 2, characterised in that an alkali chlorite and/or an earth alkali chlorite is used as chloride.

4. A method according to Claims 1-3, characterised in that the aqueous, sulphate-ion-containing solution has a pH value $\leq 1$.

5. A method according to Claims 1-5, characterised in that mineralised water is used for the production of the aqueous, sulphate-ion-containing solution.

6. A method according to Claims 1-5, characterised in that a water-soluble phosphate is added to the end product.

7. The use of aqueous chlorite solution obtained by the method according to Claims 1-6 as a biocide, especially for the external treatment of skin diseases and irritations, and for water purification.

## Revendications

1. Procédé de fabrication d'une solution aqueuse modifiée de chlorite, stabilisée par un composé peroxy, et rendue alcaline, caractérisé par le fait qu'une solution aqueuse contenant des ions sulfate et présentant un pH $\leq 3$ est mélangée à un composé peroxy, qui y est stable, qui donne dans le produit fini une concentration en produit composé peroxy d'environ 0,001 à 0,01 mole, et cette solution est ensuite mélangée à la solution aqueuse alcaline d'un chlorite d'une quantité telle que le pH est ajusté à une valeur supérieure à 7,0, en particulier entre 7,5 et 8,0.

2. Procédé selon la revendication 1, caractérisé par le fait que l'on utilise comme composé peroxy un composé peroxy anorganique sous forme de peroxyde d'hydrogène, un persulfate, un percarbonate, un perborate ou un peroxyde d'un métal alcalin ou alcalinoterreux.

3. Procédé selon l'une des revendications 1 ou 2, caractérisé par le fait que l'on utilise comme chlorite un chlorite de métal alcalin ou alcalinoterreux.

4. Procédé selon les revendications 1 à 3, caractérisé par le fait que la solution aqueuse contenant des ions sulfate a un pH $\leq 1$.

5. Procédé selon les revendications 1 à 4, caractérisé par le fait que pour produire la solution aqueuse contenant des ions sulfate, on utilise de l'eau minéralisée.

6. Procédé selon les revendications 1 à 5, caractérisé par le fait que l'on ajoute un phosphate soluble dans l'eau au produit terminé.

7. Utilisation de la solution aqueuse de chlorite, obtenue par le procédé selon les revendications 1 à 5, comme biocide, en particulier pour le traitement externe de maladies et d'irritation de la peau ainsi que pour une préparation aqueuse.